# EUROPEAN PATENT SPECIFICATION

(11) **EP 1 984 313 B1**
(45) Date of publication and mention of the grant of the patent: **15.07.2020**
(21) Application number: 06849016.8
(22) Date of filing: 21.12.2006
(51) Int. Cl.: C01B 39/48, B01J 29/70

(54) **MOLECULAR SIEVE SSZ-74 COMPOSITION OF MATTER**
MOLEKULARSIEB-SSZ-74-STOFFZUSAMMENSETZUNG
COMPOSITION DE TAMIS MOLECULAIRE SSZ- 74

(30) Priority: 28.12.2005 US 754855 P; 28.12.2005 US 754811 P; 28.12.2005 US 754858 P; 28.12.2005 US 754859 P; 28.12.2005 US 754865 P; 28.12.2005 US 754866 P; 28.12.2005 US 754867 P; 28.12.2005 US 754868 P; 28.12.2005 US 754869 P; 28.12.2005 US 755012 P
(43) Date of publication of application: 29.10.2008
(73) Proprietor: Chevron U.S.A. Inc., San Ramon, CA 94583 (US)
(72) Inventor: ZONES, Stacey, I., San Francisco, CA 94122 (US); BURTON, Allen, W., Richmond, CA 94804 (US); ONG, Kenneth, El Cerrito, CA 94530 (US)
(74) Representative: Haseltine Lake Kempner LLP
(86) International application number: PCT/US2006/049120
(87) International publication number: WO 2007/079038

(56) References cited:
- EP-A2- 0 031 255
- US-A- 4 420 467
- US-A- 4 537 757
- US-A- 4 891 458
- US-A- 5 254 684
- US-A- 5 817 878
- US-A- 6 103 653
- US-A- 6 136 290
- US-A1- 2005 042 169
- US-B1- 6 605 267

## Description

### Field of the Invention

The present invention relates to new crystalline molecular sieve SSZ-74, a method for preparing SSZ-74 using a hexamethylene-1,6-bis-(N-methyl-N-pyrrolidinium) dication as a structure directing agent ("SDA") .

### State of the Art

Because of their unique sieving characteristics, as well as their catalytic properties, crystalline molecular sieves and zeolites are especially useful in applications such as hydrocarbon conversion, gas drying and separation. Although many different crystalline molecular sieves have been disclosed, there is a continuing need for new molecular sieves with desirable properties for gas separation and drying, hydrocarbon and chemical conversions, and other applications. New molecular sieves may contain novel internal pore architectures, providing enhanced selectivities in these processes.

### SUMMARY OF THE INVENTION

The present invention is directed to a crystalline molecular sieve according to claim 1.

### Composition of Matter and Synthesis

There is described a crystalline molecular sieve having a mole ratio greater than 100 of (1) silicon oxide to (2) aluminum oxide, and having, after calcination, the X-ray diffraction lines of Table II.

There is described such a crystalline molecular sieve having a composition comprising, as synthesized and in the anhydrous state, in terms of mole ratios the following:

| | |
|---|---|
| SiO₂ / X_{c}O_{d} | greater than 100 |
| M_{2/n} / SiO₂ | 0 - 0.03 |
| Q / SiO₂ | 0.30 - 0.70 |
| F / SiO₂ | 0.30 - 0.70 |

wherein X is aluminum,
c is 2; d is 3,
M is an alkali metal cation, alkaline earth metal cation or mixtures thereof; n is the valence of M (i.e., 1 or 2); Q is a hexamethylene-1,6-bis-(N-methyl-N-pyrrolidinium) dication and F is fluoride.

There is described a method of preparing a crystalline material, said method comprising contacting under crystallization conditions a source(s) of (1) silicon oxide, (2) a source(s) of aluminum oxide, (3) fluoride ions and (4) a structure directing agent comprising a hexamethylene-1,6-bis-(N-methyl-N-pyrrolidinium) dication.

There is described such a method wherein the crystalline material has, after calcination, the X-ray diffraction lines of Table II.

There is described such a method of preparing a crystalline material which uses a reaction mixture comprising (in terms of mole ratios), the following:

| | |
|---|---|
| SiO₂ / XₐO_{b} | 100/1 and greater |
| OH- / SiO₂ | 0.20 - 0.80 |
| Q / SiO₂ | 0.20 - 0.80 |
| M_{2/n} / SiO₂ | 0 - 0.04 |
| H₂O / SiO₂ | 2 - 10 |
| HF / SiO₂ | 0.20 - 0.80 |

wherein X is aluminum,
a is 2, b is 3,
M is an alkali metal cation, alkaline earth metal cation or mixtures thereof; n is the valence of M (i.e., 1 or 2); and Q is a hexamethylene-1,6-bis-(N-methyl-N-pyrrolidinium) dication.

### BRIEF DESCRIPTION OF THE DRAWING

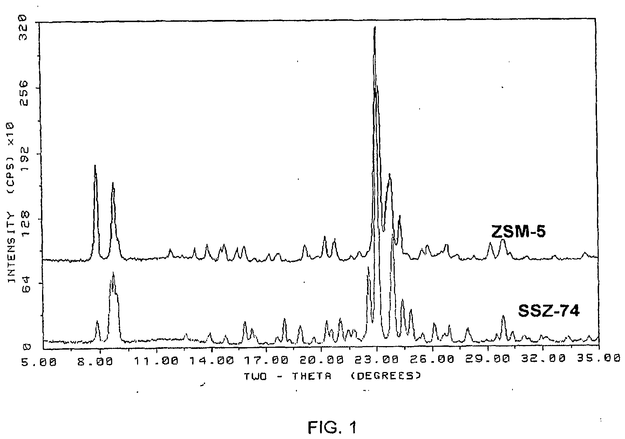

Figure 1 shows a comparison of two X-ray diffraction patterns, the top one being ZSM-5 and the bottom one being SSZ-74.

### DETAILED DESCRIPTION OF THE INVENTION

The present invention comprises a molecular sieve designated herein "molecular sieve SSZ-74" or simply "SSZ-74".

In preparing SSZ-74, a hexamethylene-1,6-bis-(N-methyl-N-pyrrolidinium) dication is used as a structure directing agent ("SDA"), also known as a crystallization template. The SDA useful for making SSZ-74 has the following structure:

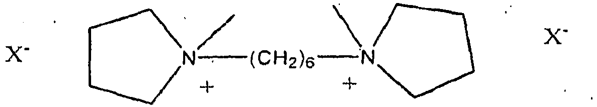

### Hexamethylene-1,6-bis-(N-methyl-N-pyrrolidinium) dication

The SDA dication is associated with anions (X⁻) which may be any anion that is not detrimental to the formation of the SSZ-74. Representative anions include halogen, e.g., fluoride, chloride, bromide and iodide, hydroxide, acetate, sulfate, tetrafluoroborate, carboxylate, and the like. Hydroxide is a typical anion, since the structure directing agent (SDA) may be used to provide hydroxide ion. Thus, it is beneficial to ion exchange, for example, a halide to hydroxide ion.

In general, SSZ-74 is prepared by contacting (1) an active source(s) of silicon oxide, and (2) an active source(s) of aluminum oxide, with the hexamethylene-1,6-bis-(N-methyl-N-pyrrolidinium) dication SDA in the presence of fluoride ion.

SSZ-74 is prepared from a reaction mixture comprising, in terms of mole ratios, the following:

**TABLE A**

| Reaction Mixture | | |
|---|---|---|
| | Embodiment 1 | Embodiment 2 |
| SiO₂ / XₐO_{b} | 100 and greater | |
| OH-/SiO₂ | 0.20 - 0.80 | 0.40 - 0.60 |
| Q / SiO₂ | 0.20 - 0.80 | 0.40 - 0.60 |
| M_{2/n}/SiO₂ | 0 - 0.04 | 0 - 0.025 |
| H₂O / SiO₂ | 2 - 10 | 3 - 7 |
| HF / SiO₂ | 0.20 - 0.80 | 0.30 - 0.60 |

where X is aluminum, a is 2, b is 3, M is an alkali metal cation, alkaline earth metal cation or mixtures thereof; n is the valence of M (i.e., 1 or 2); Q is a hexamethylene-1,6-bis-(N-methyl-N-pyrrolidinium) dication and F is fluoride.

As noted above, the SiO₂ / XₐO_{b} mole ratio in the reaction mixture is 100 and greater. This means that the SiO₂ / XₐO_{b} mole ratio can be infinity, i.e., there is no XₐO_{b} in the reaction mixture. This results in a version of SSZ-74 that is essentially all silica. As used herein, "essentially all silicon oxide" or "essentially all-silica" means that the molecular sieve's crystal structure is comprised of only silicon oxide or is comprised of silicon oxide and only trace amounts of other oxides, such as aluminum oxide, which may be introduced as impurities in the source of silicon oxide.

An example of the source of silicon oxide is tetraethyl orthosilicate. An example of the source of aluminum oxide is LZ-210 zeolite (a type of Y zeolite).

In practice, SSZ-74 is prepared by a process comprising:
(a) preparing an aqueous solution containing (1) a source(s) of silicon oxide, (2) a source(s) of aluminum oxide, (3) a source of fluoride ion and (4) a hexamethylene-1,6-bis-(N-methyl-N-pyrrolidinium) dication having an anionic counterion which is not detrimental to the formation of SSZ-74;
(b) maintaining the aqueous solution under conditions sufficient to form crystals of SSZ-74; and
(c) recovering the crystals of SSZ-74.

The reaction mixture is maintained at an elevated temperature until the crystals of the SSZ-74 are formed. The hydrothermal crystallization is usually conducted under autogenous pressure, at a temperature between 100°C and 200°C, for example between 135°C and 180°C. The crystallization period is typically greater than 1 day, for example from about 3 days to about 20 days. The molecular sieve may be prepared using mild stirring or agitation.

During the hydrothermal crystallization step, the SSZ-74 crystals can be allowed to nucleate spontaneously from the reaction mixture. The use of SSZ-74 crystals as seed material can be advantageous in decreasing the time necessary for complete crystallization to occur. In addition, seeding can lead to an increased purity of the product obtained by promoting the nucleation and/or formation of SSZ-74 over any undesired phases. When used as seeds, SSZ-74 crystals are added in an amount between 0.1 and 10% of the weight of the first tetravalent element oxide, e.g. silica, used in the reaction mixture.

Once the molecular sieve crystals have formed, the solid product is separated from the reaction mixture by standard mechanical separation techniques such as filtration. The crystals are water-washed and then dried, e.g., at 90°C to 150°C for from 8 to 24 hours, to obtain the as-synthesized SSZ-74 crystals. The drying step can be performed at atmospheric pressure or under vacuum.

SSZ-74 as prepared has the X-ray diffraction lines of Table I below. SSZ-74 has a composition, as synthesized (i.e., prior to removal of the SDA from the SSZ-74) and in the anhydrous state, comprising the following (in terms of mole ratios):

| | |
|---|---|
| SiO₂ / X_{c}O_{d} | greater than 100 |
| M_{2/n} / SiO₂ | 0 - 0.03 |
| Q / SiO₂ | 0.30 - 0.70 |
| F / SiO₂ | 0.30 - 0.70 |

wherein X is aluminum,
c is 2; d is 3, M is an alkali metal cation, alkaline earth metal cation or mixtures thereof; n is the valence of M (i.e., 1 or 2); Q is a hexamethylene-1,6-bis-(N-methyl-N-pyrrolidinium) dication and F is fluoride.

SSZ-74 is characterized by its X-ray diffraction pattern. SSZ-74, as-synthesized, has a crystalline structure whose X-ray powder diffraction pattern exhibits the characteristic lines shown in Table I.

**TABLE I**

| As-Synthesized SSZ-74 | | |
|---|---|---|
| 2 Theta^{(a)} | d-spacing (Angstroms) | Relative Integrated Intensity (%)^{(b)} |
| 7.95 | 11.11 | W |
| 8.68 | 10.18 | M |
| 8.85 | 9.98 | W-M |
| 9.02 | 9.80 | W |
| 22.69 | 3.92 | W-M |
| 23.14 | 3.84 | VS |
| 24.01 | 3.70 | M |
| 24.52 | 3.63 | W |
| 24.93 | 3.57 | W |
| 29.95 | 2.98 | W |

| | | |
|---|---|---|
| ^{(a)} ± 0.1 ^{(b)} The X-ray patterns provided are based on a relative intensity scale in which the strongest line in the X-ray pattern is assigned a value of 100: W(weak) is less than 20; M(medium) is between 20 and 40; S(strong) is between 40 and 60; VS(very strong) is greater than 60. | | |

Table IA below shows the X-ray powder diffraction lines for as-synthesized SSZ-74 including actual relative intensities.

**TABLE IA**

| As-Synthesized SSZ-74 | | |
|---|---|---|
| 2 Theta^{(a)} | d-spacing (Angstroms) | Intensity |
| 7.95 | 11.11 | 7.9 |
| 8.68 | 10.18 | 21.1 |
| 8.85 | 9.98 | 18.7 |
| 9.02 | 9.80 | 11.3 |
| 11.30 | 7.82 | 0.4 |
| 12.70 | 6.96 | 1.8 |
| 13.98 | 6.33 | 2.4 |
| 14.77 | 5.99 | 0.5 |
| 14.85 | 5.96 | 2.1 |
| 15.93 | 5.56 | 6.3 |
| 16.30 | 5.43 | 4.6 |
| 16.50 | 5.37 | 1.8 |
| 17.05 | 5.20 | 0.8 |
| 17.41 | 5.09 | 0.1 |
| 17.71 | 5.00 | 2.0 |
| 18.09 | 4.90 | 7.4 |
| 18.38 | 4.82 | 0.7 |
| 18.89 | 4.69 | 0.9 |
| 18.96 | 4.68 | 4.4 |
| 19.69 | 4.51 | 1.8 |
| 20.39 | 4.35 | 5.1 |
| 20.63 | 4.30 | 4.2 |
| 21.12 | 4.20 | 7.7 |
| 21.55 | 4.12 | 5.4 |
| 21.75 | 4.08 | 0.5 |
| 21.80 | 4.07 | 1.4 |
| 21.88 | 4.06 | 2.1 |
| 21.96 | 4.04 | 1.5 |
| 22.17 | 4.01 | 0.8 |
| 22.69 | 3.92 | 18.9 |
| 23.14 | 3.84 | 100.0 |
| 23.89 | 3.72 | 9.4 |
| 24.01 | 3.70 | 25.6 |
| 24.52 | 3.63 | 13.7 |
| 24.68 | 3.60 | 2.1 |
| 24.93 | 3.57 | 11.3 |
| 25.09 | 3.55 | 0.9 |
| 25.37 | 3.51 | 1.7 |
| 25.57 | 3.48 | 2.7 |
| 26.20 | 3.40 | 5.5 |
| 26.31 | 3.38 | 0.8 |
| 26.67 | 3.34 | 2.0 |
| 26.76 | 3.33 | 1.0 |
| 26.82 | 3.32 | 0.9 |
| 27.01 | 3.30 | 3.4 |
| 27.05 | 3.29 | 0.8 |
| 27.48 | 3.24 | 0.8 |
| 27.99 | 3.19 | 4.2 |
| 28.18 | 3.16 | 0.8 |
| 28.78 | 3.10 | 0.6 |
| 29.03 | 3.07 | 0.7 |
| 29.31 | 3.04 | 0.9 |
| 29.58 | 3.02 | 2.4 |
| 29.95 | 2.98 | 9.6 |
| 30.44 | 2.93 | 3.7 |
| 31.09 | 2.87 | 3.1 |
| 31.36 | 2.85 | 0.8 |
| 31.98 | 2.80 | 2.2 |
| 32.23 | 2.78 | 1.7 |
| 32.37 | 2.76 | 0.6 |
| 32.64 | 2.74 | 1.5 |
| 33.03 | 2.71 | 0.1 |
| 33.34 | 2.69 | 1.0 |
| 33.47 | 2.68 | 1.3 |
| 34.08 | 2.63 | 0.7 |
| 34.55 | 2.59 | 1.8 |
| 34.73 | 2.58 | 0.4 |

| | | |
|---|---|---|
| ^{(a)} ± 0.1 | | |

After calcination, the X-ray powder diffraction pattern for SSZ-74 exhibits the characteristic lines shown in Table II below.

**TABLE II**

| Calcined SSZ-74 | | |
|---|---|---|
| 2 Theta^{(a)} | d-spacing (Angstroms) | Relative Inteqrated Intensity (%) |
| 7.98 | 11.07 | M |
| 8.70 | 10.16 | VS |
| 8.89 | 9.93 | S |
| 9.08 | 9.74 | S |
| 14.02 | 6.31 | W |
| 14.93 | 5.93 | M |
| 16.03 | 5.52 | M |
| 23.26 | 3.82 | VS |
| 23.95 | 3.71 | W |
| 24.08 | 3.69 | M |

| | | |
|---|---|---|
| ^{(a)} ± 0.1 | | |

Table IIA below shows the X-ray powder diffraction lines for calcined SSZ-74 including actual relative intensities.

**TABLE IIA**

| Calcined SSZ-74 | | |
|---|---|---|
| 2 Theta^{(a)} | d-spacing (Angstroms) | Relative Integrated Intensity (%) |
| 7.98 | 11.07 | 34.9 |
| 8.70 | 10.16 | 86.8 |
| 8.89 | 9.93 | 40.2 |
| 9.08 | 9.74 | 47.0 |
| 9.66 | 9.15 | 1.0 |
| 11.26 | 7.85 | 0.4 |
| 11.34 | 7.80 | 0.5 |
| 12.76 | 6.93 | 1.1 |
| 13.26 | 6.67 | 4.6 |
| 14.02 | 6.31 | 13.4 |
| 14.93 | 5.93 | 20.9 |
| 16.03 | 5.52 | 23.5 |
| 16.39 | 5.40 | 4.3 |
| 16.61 | 5.33 | 4.4 |
| 17.12 | 5.18 | 3.0 |
| 17.80 | 4.98 | 2.8 |
| 18.19 | 4.87 | 7.6 |
| 19.05 | 4.66 | 1.9 |
| 19.74 | 4.49 | 0.4 |
| 20.44 | 4.34 | 3.0 |
| 20.75 | 4.28 | 3.4 |
| 21.19 | 4.19 | 7.7 |
| 21.67 | 4.10 | 4.1 |
| 21.99 | 4.04 | 5.8 |
| 22.68 | 3.92 | 3.7 |
| 22.79 | 3.90 | 9.5 |
| 23.26 | 3.82 | 100.0 |
| 23.95 | 3.71 | 14.2 |

| | | |
|---|---|---|
| ^{(a)} ±0.1 | | |

The X-ray powder diffraction patterns were determined by standard techniques. The radiation was the K-alpha/doublet of copper. The peak heights and the positions, as a function of 2θ where θ is the Bragg angle, were read from the relative intensities of the peaks, and d, the interplanar spacing in Angstroms corresponding to the recorded lines, can be calculated.

The variation in the scattering angle (two theta) measurements, due to instrument error and to differences between individual samples, is estimated at ± 0.1 degrees.

Representative peaks from the X-ray diffraction pattern of calcined SSZ-74 are shown in Table II. Calcination can result in changes in the intensities of the peaks as compared to patterns of the "as-made" material, as well as minor shifts in the diffraction pattern.

Crystalline SSZ-74 can be used as-synthesized, but typically will be thermally treated (calcined). Usually, it is desirable to remove the alkali metal cation (if any) by ion exchange and replace it with hydrogen; ammonium, or any desired metal ion.

SSZ-74 can be formed into a wide variety of physical shapes. Generally speaking, the molecular sieve can be in the form of a powder, a granule, or a molded product, such as extrudate having a particle size sufficient to pass through a 2-mesh (Tyler) screen and be retained on a 400-mesh (Tyler) screen. In cases where the catalyst is molded, such as by extrusion with an organic binder, the SSZ-74 can be extruded before drying, or, dried or partially dried and then extruded.

SSZ-74 can be composited with other materials resistant to the temperatures and other conditions employed in organic conversion processes. Such matrix materials include active and inactive materials and synthetic or naturally occurring zeolites as well as inorganic materials such as clays, silica and metal oxides. Examples of such materials and the manner in which they can be used are disclosed in U.S. Patent No. 4,910,006, issued May 20, 1990 to Zones et al., and U.S. Patent No. 5,316,753, issued May 31, 1994 to Nakagawa.

### EXAMPLES

The following examples demonstrate but do not limit the present invention.

### Example 1

### Synthesis of Hexamethylene-1,6-bis-(N-methyl-N-pyrrolidinium) dication SDA

In 50 ml of acetone was dissolved 5ml (48 mmoles) of N-methyl pyrrolidine. 4.9 Grams of 1,6 dibromohexane (20 mmoles) were added and the resulting mixture was stirred at room temperature for three days. Solids formed and were collected by filtration and washed with ether and kept in a vacuum oven. Then 3.71 grams of the dried solid was mixed into 18.7 grams of water and 9.57 grams of AG1-X8 resin for exchange to the OH form. The exchange was run overnight and then the solution was collected and titrated.

### Example 2

### Synthesis of All-Silica SSZ-74

6.4 Grams of the solution from Example 1 (3 mmoles) was mixed in a tared Teflon cup with 1.26 grams of tetraethyl orthosilicate and then allowed to evaporate (in a hood) for several days as hydrolysis occurred. A second reaction was set up the same way. After evaporation to the appearance of dryness, one reaction was given 0.20 gram of water and mixed. The second was given 0.60 gram of water and the same treatment ensued 0.125 Gram of about 50% HF was carefully added to each reaction mixture and the contents were stirred with a plastic spatula and a thick gel formed. In the first case the H2O/SiO2 ratio was now roughly 3.5 and it was 7.0 in the second case. The materials were heated to 150° C and at 43 RPM in tumbled Parr reactors placed in a Blue M convection heating oven. The reactions were cooled and opened in 6 day periods with a small amount examined by Scanning Electron Microscopy to determine if crystals had formed. After 22 days there was crystalline material in both and the solids were collected (filtration) and washed with copious amounts of water, air dried and then examined by X-ray diffraction (XRD). The product in both cases was SSZ-74.

### Example 3

### Calcination of SSZ-74

The products from both reactions in Example 2 were calcined in stages and in air to 595° C to remove the organic content. The materials were found to be stable and the XRD patterns showed the relationship to the as-made SSZ-74.

### Example 4

### Adsorption of 2,2-Dimethylbutane

The calcined material of Example 3 was then tested for the uptake of the hydrocarbon 2,2-dimethylbutane. This adsorbate does not enter small pore zeolites (8-ring portals) and sometimes is hindered in entering intermediate pore zeolites like ZSM-5. The SSZ-74 showed a profile more characteristic of intermediate pore materials (as contrasted to Y zeolite, a large pore materia), showing steady gradual uptake of the adsorbate.

SSZ-74 was shown to adsorb about 0.08 cc/gram after 3 hours of exposure to the 2,2 dimethyl butane adsorbate using a pulsed mode. This value compares with an analysis for ZSM-5 zeolite which gives a value closer to 0.07cc/gm at the same point in time under the same experimental conditions. This would indicate that the pores of SSZ-74 are at least 10-rings

### Example 5

### Synthesis of Aluminosilicate SSZ-74

The synthesis parameters of Example 2 were repeated except for the following changes. (1) 0.04 gram of Y zeolite material LZ-210 was added as a potential contributor of Al; (2) the initial H2O/SiO2 ratio for the synthesis was adjusted to 5; (3) seeds of a successful SSZ-74 product were added; and (4) the reaction was run at 170° C. After 9 days there was crystalline material which was SSZ-74 when worked up and analyzed by XRD. The solids were calcined then as in Example 3.

### Example 6

### Constraint Index

0.12 grams of the material from Example 5, in a 20-40 pelleted and meshed range, was loaded into a stainless steel reactor and run in a Constraint Index test (50/50 n-hexane/3-methylpentane). The normal feed rate was used (8 µl/min.) and the test was run at 700° F after the catalyst had been dried in the reactor to near 1000° F. Helium flow was used. At 10 minutes on-stream nearly 30% of the feed was being converted with about equal amounts of each reactant. The selectivity did not change as the catalyst fouled to half the conversion at 100 minutes. The pores of the active SSZ-74 were at least intermediate in size.

### Example 7

### Synthesis of Aluminosilicate SSZ-74

Three mMoles of SDA solution and 1.26 grams (6 mMoles) of tetraethylorthosilicate were combined in a Teflon cup for a Parr reactor. The contents were allowed to react and then most of the water and then the ethanol by-product were allowed to evaporate in a hood over several days. Once the H2O/SiO2 ratio was about 5, from the evaporation, 0.04 grams of LZ-210 zeolite were added (LZ-210 is a Y zeolite which has been treated with (NH₄⁺)₂SiF₆ to provide some de-alumination). A few mg of seeds of SSZ-74 were added in the as-made state. Lastly, 0.132 gram of 50% HF was added and the reactor was closed up and heated at 170°C, 43 RPM, for six days. A sample of the cooled reaction product showed nicely crystalline material in an electron microscope. The reaction contents were worked up and dried. Analysis by X-ray diffraction showed the product to be molecular sieve SSZ-74.

The sample was calcined (in air to 595°C ) and then pelleted and meshed (20-40) and run in a standard Constraint Index test. At 700°F the initial conversion was 28% with a Cl value of 1.1. With time-on-stream the catalyst showed a steady deactivation while the Cl value did not change much.

## Claims

1. A crystalline molecular sieve having a mole ratio greater than 100 of (1) silicon oxide to (2) aluminum oxide and having, after calcination, the following X-ray diffraction lines:
| 2 Theta^{(a)} | d-spacing (Angstroms) | Relative Integrated Intensity (%) |
|---|---|---|
| 7.98 | 11.07 | M |
| 8.70 | 10.16 | VS |
| 8.89 | 9.93 | S |
| 9.08 | 9.74 | S |
| 14.02 | 6.31 | W |
| 14.93 | 5.93 | M |
| 16.03 | 5.52 | M |
| 23.26 | 3.82 | VS |
| 23.95 | 3.71 | W |
| 24.08 | 3.69 | M |
| | | |
|---|---|---|
| ^{(a)} ± 0.1 | | |

2. The molecular sieve of claim 1 which is comprised of essentially all silicon oxide.

## Patentansprüche

1. Kristallines Molekularsieb mit einem Molverhältnis größer als 100 von (1) Siliziumoxid zu (2) Aluminiumoxid und mit, nach Kalzinierung, den folgenden Röntgenbeugungslinien:
| 2 Theta^{(a)} | d-Abstand (Angström) | Relative Integrierte Intensität (%) |
|---|---|---|
| 7,98 | 11,07 | M |
| 8,70 | 10,16 | VS |
| 8,89 | 9,93 | S |
| 9,08 | 9,74 | S |
| 14,02 | 6,31 | W |
| 14,93 | 5,93 | M |
| 16,03 | 5,52 | M |
| 23,26 | 3,82 | VS |
| 23,95 | 3,71 | W |
| 24,08 | 3,69 | M |
| | | |
|---|---|---|
| ^{(a)} ± 0,1 | | |

2. Molekularsieb gemäß Anspruch 1, das im Wesentlichen ganz aus Siliziumoxid besteht.

## Revendications

1. Un tamis moléculaire cristallin ayant un rapport molaire supérieur à 100 de (1) l'oxyde de silicium à (2) l'oxyde d'aluminium et ayant, après calcination, les raies de diffraction des rayons X suivantes:
| 2 Thêta^{(a)} | Espacement-d (Angstroms) | Intensité Relative Intégrée (%) |
|---|---|---|
| 7,98 | 11,07 | M |
| 8,70 | 10,16 | VS |
| 8,89 | 9,93 | S |
| 9,08 | 9,74 | S |
| 14,02 | 6,31 | W |
| 14,93 | 5,93 | M |
| 16,03 | 5,52 | M |
| 23,26 | 3,82 | VS |
| 23,95 | 3,71 | W |
| 24,08 | 3,69 | M |
| | | |
|---|---|---|
| ^{(a)} ± 0,1 | | |

2. Le tamis moléculaire selon la revendication 1 qui est composé essentiellement entièrement d'oxyde de silicium.
